# EUROPEAN PATENT APPLICATION

(11) **EP 4 502 583 A1**
(43) Date of publication of application: **05.02.2025**
(21) Application number: 23779557.0
(22) Date of filing: 14.03.2023
(51) Int. Cl.: G01N 27/00, G01N 1/00, G01N 27/06, G01N 27/22, G05B 23/02

(54) **INSPECTION EQUIPMENT CONDITION MONITORING SYSTEM AND PROGRAM**

(30) Priority: 28.03.2022 JP 2022052519
(71) Applicant: KYB Corporation, Minato-ku, Tokyo 105-5128 (JP)
(72) Inventor: YOSHIDA, Takahiro, Tokyo 105-5128 (JP); KAMEDA, Yukinori, Tokyo 105-5128 (JP)
(74) Representative: Grünecker Patent- und Rechtsanwälte PartG mbB
(86) International application number: PCT/JP2023/009856
(87) International publication number: WO 2023/189538

(57) **Abstract**

The state monitoring system (100) of the inspection equipment includes: the fluid property sensor (10) provided on the inspection equipment (5) for performing the inspection by operating the fluid pressure cylinder (1), serving as the inspection target, by supplying and discharging the inspection fluid to and from the fluid pressure cylinder (1) to be sequentially exchanged with other fluid pressure cylinder (1), the fluid property sensor (10) being configured to detect the property of the inspection fluid; the exchange timing acquisition unit (33) configured to acquire the timing at which the fluid pressure cylinder (1) is exchanged; and the identification unit (34) configured to identify, when the detected value from the fluid property sensor (10) is changed so as to exceed the predetermined value, the fluid pressure cylinder (1) corresponding to the timing at which the change has caused on the basis of the acquisition result from the exchange timing acquisition unit (33).

## Description

### TECHNICAL FIELD

The present invention relates to a state monitoring system and a program of inspection equipment.

### BACKGROUND ART

WO2019/021502A1 discloses an oil diagnostic system including a controller for diagnosing a machine on the basis of sensor information including viscosity, density, and dielectric constant of oil obtained via an oil sensor mounted on the machine. The controller includes an abnormality determination unit that determines abnormalities of the oil, and the abnormality determination unit determines the abnormalities of the oil on the basis of temporal changes in the sensor information.

### SUMMARY OF INVENTION

Here, consideration is given to apply an oil diagnostic system as described in WO2019/021502A1 to inspection equipment that sequentially performs inspection of inspection targets, such as actuators, etc., by using an inspection fluid, such as oil, etc. In the inspection equipment, the inspection is performed by operating the inspection target by supplying and discharging the inspection fluid to and from the inspection targets that are exchanged sequentially. In such an inspection equipment, if a contaminant is present in the inspection target, the contaminant is mixed into the inspection fluid to be supplied to the inspection target from the inspection equipment during the inspection. The inspection fluid containing the contaminant is then discharged from the inspection target to the inspection equipment. As described above, if the contaminant is present in the inspection target, deterioration of the inspection fluid of the inspection equipment progresses more significantly than under normal inspection state. In addition, when the contaminant is present in a certain inspection target, there is a possibility that the contaminant is also present in following inspection target, which has been manufactured in the same environment as the inspection target, in a similar manner. In such a case, the deterioration of the inspection fluid progresses even further.

With the oil diagnostic system as described in WO2019/021502A1, while it is possible to identify the time at which the deterioration of the inspection fluid is caused because the deterioration of the fluid is always determined on the basis of the temporal changes of the sensor information, it is not possible to identify the inspection target that was being inspected when the deterioration of the inspection fluid progressed significantly. In other words, it is not possible to identify the inspection target that caused the deterioration of the inspection fluid. Consequently, it is difficult to identify the cause of the deterioration of the inspection fluid, and it is difficult to prevent the deterioration of the inspection fluid.

An object of the present invention is to effectively prevent deterioration of an inspection fluid of inspection equipment that performs an inspection by operating an inspection target by supplying and discharging an inspection fluid to and from the inspection targets, which are exchanged sequentially.

According to one aspect of the present invention, a state monitoring system of inspection equipment includes: a fluid property sensor provided on the inspection equipment for performing inspection by operating an inspection target by supplying and discharging inspection fluid to and from the inspection target, the inspection target being sequentially exchanged with other inspection targets, and the fluid property sensor being configured to detect a property of the inspection fluid; an exchange timing acquisition unit configured to acquire a timing at which the inspection target is exchanged; and an identification unit configured to identify, when a detected value from the fluid property sensor is changed so as to exceed a predetermined value, an inspection target corresponding to a timing at which the change has caused based on an acquisition result from the exchange timing acquisition unit.

According to another aspect of the present invention, a program for causing a computer to execute processing of a detected value input from a fluid property sensor for detecting a property of inspection fluid by being provided on inspection equipment, the inspection equipment performing an inspection by operating an inspection target by supplying and discharging the inspection fluid to and from the inspection target, and the inspection target being sequentially exchanged with other inspection targets, the program causes the computer to: acquire a timing at which the inspection target is exchanged; and identify, when the detected value is changed so as to exceed a predetermined value, an inspection target corresponding to a timing at which the change has caused based on the timing at which the inspection target is exchanged.

### BRIEF DESCRIPTION OF DRAWINGS

[FIG. 1] FIG. 1 is a schematic view showing an inspection of actuators by inspection equipment.
[FIG. 2] FIG. 2 is a schematic view of a state monitoring system of the inspection equipment according to an embodiment of the present invention.
[FIG. 3] FIG. 3 is a diagram showing a relationship between an elapsed time and an electrical characteristic value of fluid during operation of the inspection equipment.
[FIG. 4] FIG. 4 is a schematic view of a state monitoring system of the inspection equipment according to a modification of the embodiment of the present invention.

### DESCRIPTION OF EMBODIMENTS

A state monitoring system of inspection equipment according to an embodiment of the present invention will be described with reference to the drawings.

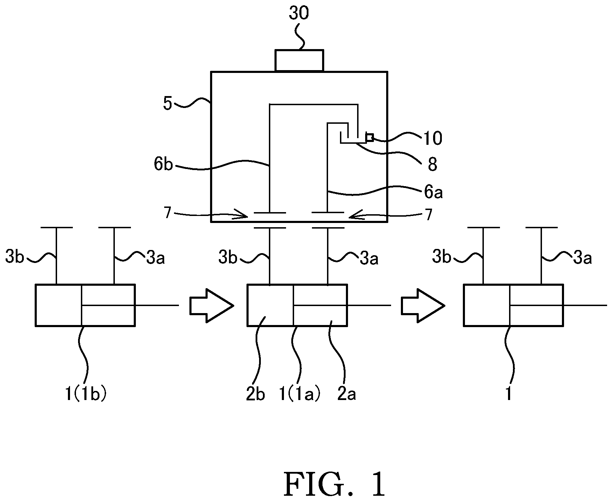

As shown in FIG. 1, the state monitoring system of the inspection equipment (hereinafter, also simply referred to as a "state monitoring system") 100 is applied to, for example, inspection equipment 5 that sequentially performs inspection of fluid pressure cylinders 1 (actuators), which are each an inspection target, by using an inspection fluid (hereinafter, also simply referred to as a "fluid"), such as oil, etc.

The inspection equipment 5 performs, for example, a post-manufacturing test of a hydraulic pump, a hydraulic motor, a hydraulic valve, a hydraulic cylinder, and so forth, as the inspection. In this embodiment, the inspection equipment 5 performs a post-manufacturing performance test of the fluid pressure cylinders 1. The inspection equipment 5 has flow paths 6a and 6b for guiding the fluid. In addition, each of the fluid pressure cylinders 1 has flow paths 3a and 3b that respectively communicate with a rod side chamber 2a and an anti-rod side chamber 2b and guide the fluid. When the fluid pressure cylinder 1 is to be inspected by the inspection equipment 5, the flow path 6a is linked to the flow path 3a of the fluid pressure cylinder 1, and the flow path 6b is linked to the flow path 3b of the fluid pressure cylinder 1, each via a coupling 7. As a result, the inspection equipment 5 is connected to the fluid pressure cylinder 1, and the fluid pressure cylinder 1 is operated by supplying the fluid to the fluid pressure cylinder 1 and by discharging the fluid from the fluid pressure cylinder 1. In this way, the inspection of the fluid pressure cylinder 1 is performed. When the inspection of a certain fluid pressure cylinder 1a is finished, the flow paths 3a and 3b of the fluid pressure cylinder 1a are disconnected from the inspection equipment 5, and the inspection equipment 5 is connected to flow paths 3a and 3b of a fluid pressure cylinder 1b, which is a following inspection target, to perform the inspection of the fluid pressure cylinder 1b. In this way, the inspection equipment 5 performs the inspection by operating the fluid pressure cylinders 1 by supplying and discharging the fluid to and from the fluid pressure cylinders 1, which are exchanged sequentially. The fluid pressure cylinder 1 may have a configuration in which the fluid pressure cylinder 1 is connected to the inspection equipment 5 without having the flow paths 3a and 3b.

In addition, the inspection equipment 5 outputs inspection history of the fluid pressure cylinder 1 to a controller 30, which will be described later. In the inspection history of the fluid pressure cylinder 1, the time at which the inspection of the fluid pressure cylinder 1 was performed and a model number, etc. of the fluid pressure cylinder 1 are associated with each other.

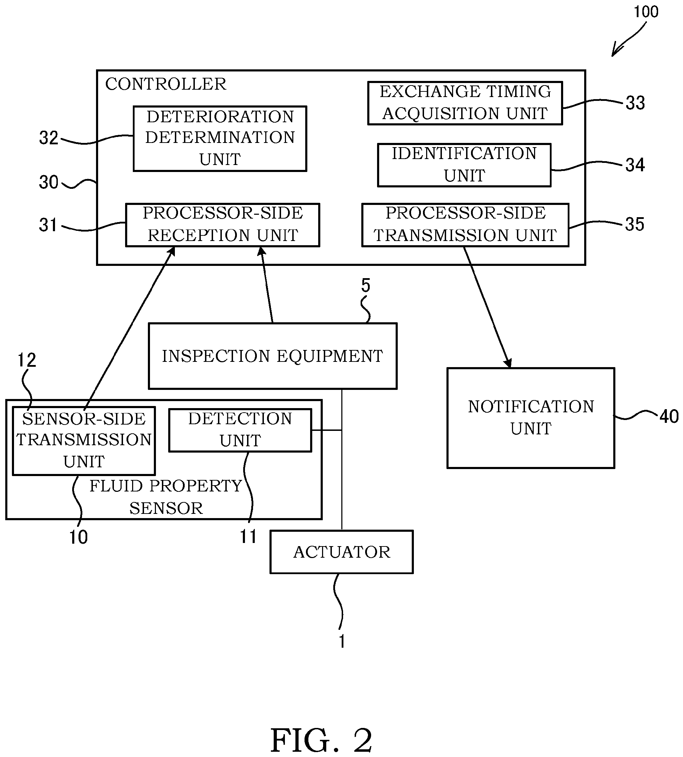

As shown in FIGs. 1 and 2, the state monitoring system 100 includes a fluid property sensor 10 that is provided on the inspection equipment 5 and detects properties of the fluid, the controller 30 that processes the information from the fluid property sensor 10, and a notification unit 40 that receives a signal from the controller 30 and provides the information in accordance with the signal. The state monitoring system 100 monitors the deterioration of the fluid of the inspection equipment 5 by using the fluid property sensor 10 and the controller 30.

The fluid property sensor 10 is provided on a tank 8 that stores the fluid of the inspection equipment 5, and in order to monitor the deterioration of the fluid, detects an electrical characteristic value, temperature, and so forth as the property of the fluid. The fluid property sensor 10 may be provided on the flow path 6a or the flow path 6b of the inspection equipment 5. The fluid property sensor 10 has a detection unit 11 that detects the property of the fluid and a sensor-side transmission unit 12 that sends the detection result from the detection unit 11 to the controller 30. In this embodiment, the detection unit 11 has a pair of electrodes (not shown). The detection unit 11 detects the electrical characteristic value such as a dielectric constant, a conductivity, and so forth of the fluid on the basis of an electrostatic capacitance and a resistance value obtained by applying a voltage to the pair of electrodes. In addition, the detection unit 11 also detects the temperature of the fluid by a temperature sensor. Because a known configuration can be adopted for the detection unit 11, detailed illustration and description of the configuration will be omitted. In addition, in the following, although a description will be given of a case in which the fluid property sensor 10 detects the dielectric constant and the conductivity as the electrical characteristic value, the electrical characteristic value to be detected by the fluid property sensor 10 is not limited to the dielectric constant and the conductivity. The sensor-side transmission unit 12 sends the electrical characteristic value and the temperature detected by the detection unit 11 to the controller 30 continuously or at constant time intervals by wireless communication.

The controller 30 is provided on the inspection equipment 5. The controller 30 has a processing unit, such as a CPU, etc., a storage device, a display, an input device, a communication device, and so forth, and a program stored in the storage device in advance is executed by the CPU to execute respective processes performed by the controller 30, which will be described below. In this embodiment, the controller 30 is a device (computer) that wirelessly communicates with the fluid property sensor 10 and the notification unit 40. The controller 30 may also be a device that communicates with the fluid property sensor 10 and the notification unit 40 via a wired communication. In addition, the controller 30 may be a server in a cloud that is provided outside the inspection equipment 5 and wirelessly communicates with the fluid property sensor 10 and the notification unit 40. As described above, the controller 30 communicates with the fluid property sensor 10 and the notification unit 40 via a network.

The controller 30 determines whether or not a maintenance of the inspection equipment 5 is required on the basis of a detected value input from the fluid property sensor 10. The controller 30 has: a processor-side reception unit 31 that receives the detected value from the fluid property sensor 10 that is sent from the sensor-side transmission unit 12 of the fluid property sensor 10; a deterioration determination unit 32 that determines the deterioration of the fluid on the basis of the detected value from the fluid property sensor 10; and a processor-side transmission unit 35 that sends the detection result from the deterioration determination unit 32 and an identification unit 34, which will be described later, to the notification unit 40. Note that these components such as the processor-side reception unit 31, the identification unit 34, which will be described later, and so forth show the respective functions of the controller 30 as virtual units, and it does not mean that they exist physically.

The detected value (the electrical characteristic value and the temperature) is input to the processor-side reception unit 31 from the fluid property sensor 10, and the inspection history of the fluid pressure cylinder 1 is input to the processor-side reception unit 31 from the inspection equipment 5.

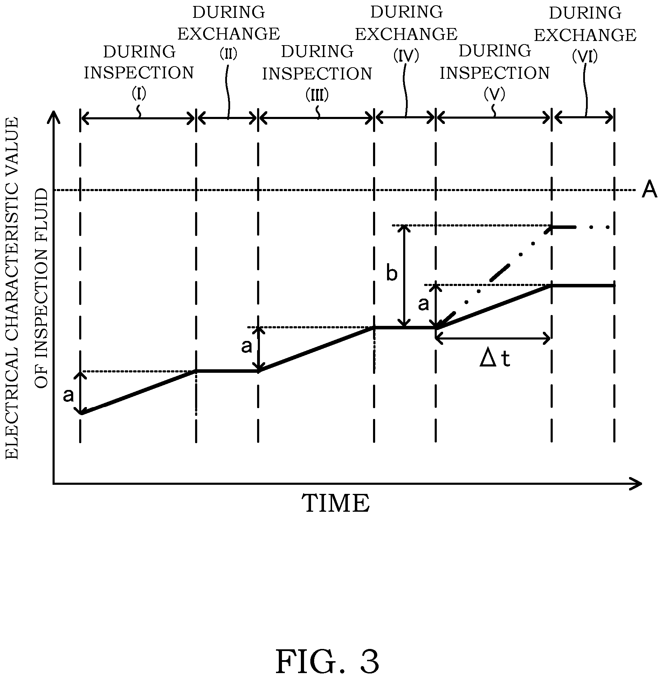

The deterioration determination unit 32 determines the deterioration of the fluid on the basis of the detected value from the fluid property sensor 10. Specifically, the deterioration determination unit 32 determines whether or not the maintenance of the inspection equipment 5, such as the fluid replacement, etc., is required on the basis of the electrical characteristic value of the fluid detected by the fluid property sensor 10. In the following, a description will be given of a case in which the replacement of the fluid is performed as the maintenance. The electrical characteristic value of the fluid (the dielectric constant and the conductivity) is increased with the deterioration of the fluid. In this embodiment, as shown with a solid line in FIG. 3, the fluid is deteriorated each time the inspection of the fluid pressure cylinder 1 is performed (sections I, III, and V shown in FIG. 3), and the electrical characteristic value of the fluid is increased. The deterioration determination unit 32 determines whether or not the electrical characteristic value of the fluid detected by the fluid property sensor 10 falls within a predetermined threshold value A (whether or not the electrical characteristic value is equal to or less than the predetermined threshold value A). When the electrical characteristic value exceeds the threshold value A, it is determined that the fluid is deteriorated and the replacement thereof is required, and a maintenance signal is output to the notification unit 40 via the processor-side transmission unit 35. Even when the electrical characteristic value falls within the threshold value A and it is determined that the maintenance is not required, a signal may be output to notify an operator of a degree of deterioration of the fluid.

Even in a state in which it is not determined that the fluid is deteriorated by the deterioration determination unit 32, as indicated by a two-dot chain line in the section V shown in FIG. 3, it is conceivable that the deterioration of the fluid progresses more significantly than under the normal inspection state during the process of the inspection of the fluid pressure cylinder 1. For example, in a case in which the environments in upstream process of the inspection by the inspection equipment 5 are different between the fluid pressure cylinder 1 that has been inspected in the section V shown in FIG. 3 and the fluid pressure cylinders 1 that have been respectively inspected in previous sections I and III, significant progression of the deterioration of the fluid of the inspection equipment 5 may occur. Specifically, in a case in which the fluid pressure cylinder 1 that has been inspected in the section V and the fluid pressure cylinders 1 that have been inspected in the sections I and III are different in model, and the manufacturing process and apparatus used for the manufacturing employed for them are different, the significant progression of the deterioration of the fluid of the inspection equipment 5 may occur. In addition, even when the fluid pressure cylinder 1 that has been inspected in the section V and the fluid pressure cylinders 1 that have been inspected in the sections I and III are of the same model, if different workers performed the operations, the significant progression of the deterioration of the fluid of the inspection equipment 5 may occur.

A cause of the significant progression of the deterioration of the fluid could be as follows: if there is an issue in the upstream process of the inspection of the fluid pressure cylinder 1 performed by the inspection equipment 5 for the fluid pressure cylinder 1 that has been inspected in the section V, and if a cutting fluid from machining, a cleaning liquid from cleaning, moisture, and so forth remained in the fluid pressure cylinder 1 as a contaminant, the contaminant would be mixed into the fluid supplied into the fluid pressure cylinder 1 from the inspection equipment 5 during the inspection. The fluid contaminated with the contaminant is then discharged from the fluid pressure cylinder 1 to the inspection equipment 5. As a result, the deterioration of the fluid of the inspection equipment 5 progresses significantly. In addition, if the state in which there is an issue in the upstream process of the inspection of the fluid pressure cylinder 1 that has been inspected in the section V is not solved, there is a possibility that the contaminant may continue to be mixed into the fluid pressure cylinders 1 that are manufactured using the same manufacturing process and apparatus as the concerned fluid pressure cylinder 1 and/or into the fluid pressure cylinders 1 that are manufactured by the same operator as the concerned fluid pressure cylinder 1. In this case, this causes further progression of the deterioration of the fluid.

Therefore, the controller 30 acquires the timing at which the fluid pressure cylinder 1 is exchanged during the inspection from the detected value input from the fluid property sensor 10. The controller 30 identifies, on the basis of the timing at which the fluid pressure cylinder 1 is exchanged, the fluid pressure cylinder 1 that has been inspected when the deterioration of the fluid progressed significantly.

The controller 30 has: an exchange timing acquisition unit 33 that acquires the timing at which the fluid pressure cylinder 1 is exchanged; and the identification unit 34 that identifies, when the detected value from the fluid property sensor 10 is changed so as to exceed a predetermined value (specifically, when the deterioration is caused such that a predetermined amount of change is exceeded), the fluid pressure cylinder 1 corresponding to the timing at which the change has caused on the basis of the acquisition result of the exchange timing acquisition unit 33.

The exchange timing acquisition unit 33 acquires the timing at which the fluid pressure cylinder 1 is exchanged from the temperature of the fluid detected by the fluid property sensor 10. During the inspection of the fluid pressure cylinder 1, the temperature of the fluid supplied to the fluid pressure cylinder 1 is increased by the heat generated during the operation of the fluid pressure cylinder 1. Therefore, the temperature of the fluid is increased during the inspection of the fluid pressure cylinder 1. On the other hand, when the fluid pressure cylinder 1 is being exchanged, the temperature of the fluid is not increased, and the temperature of the fluid is lowered. Thus, the exchange timing acquisition unit 33 determines whether or not the temperature of the fluid detected by the fluid property sensor 10 falls within a predetermined threshold value (whether or not it is equal to or less than a predetermined threshold value). Here, "a predetermined threshold value" is set so as not to include lower temperature of the fluid at the time of exchange of the fluid pressure cylinder 1. When the temperature of the fluid becomes lower than the threshold value, it is detected that the fluid pressure cylinder 1 is being exchanged, and the detected exchange timing is output to the identification unit 34.

When the detected value from the fluid property sensor 10 is changed so as to exceed the predetermined value, the identification unit 34 identifies the fluid pressure cylinder 1 corresponding to the timing at which the change has caused on the basis of the acquisition result from the exchange timing acquisition unit 33. Specifically, as indicated by the two-dot chain line in the section V shown in FIG. 3, when the amount of change of the detected value from the fluid property sensor 10 per unit time (b/Δt shown in FIG. 3) is larger than the amount of change per unit time in the normal inspection state (a/Δt shown in FIG. 3), the identification unit 34 identifies the fluid pressure cylinder 1 corresponding to the timing at which the change has caused. Here, the "predetermined value" is set so as to be larger than the amount of change of the detected value from the fluid property sensor 10 per unit time in the normal inspection state (a/Δt shown in FIG. 3). In other words, the phrase "the detected value from the fluid property sensor 10 is changed so as to exceed the predetermined value" means that the deterioration of the fluid has progressed during the inspection more significantly than under the normal inspection state due to the contaminant contained in the fluid pressure cylinder 1.

The identification unit 34 associates the detected value from the fluid property sensor 10 with the timing at which the fluid pressure cylinder 1 is exchanged, and when the detected value from the fluid property sensor 10 is changed so as to exceed the predetermined value, the identification unit 34 identifies the timing at which the fluid pressure cylinder 1 has exchanged before and after the change. Specifically, for the section V shown in FIG. 3, in which the detected value from the fluid property sensor 10 is changed greatly, the identification unit 34 identifies the sections IV and VI which are each the timing at which the fluid pressure cylinder 1 is exchanged before and after the change. Then, based on the concerned timing and the inspection history of the fluid pressure cylinder 1 input to the controller 30, the identification unit 34 identifies the model number of the fluid pressure cylinder 1 that has been being inspected when the detected value from the fluid property sensor 10 is changed (in other words, during the identified timing). The identification unit 34 outputs, to the notification unit 40, an identification signal including information indicating that the deterioration of the fluid has progressed significantly in the inspection of the fluid pressure cylinder 1 and the information on the model number of the identified fluid pressure cylinder 1.

The notification unit 40 is, for example, a lamp or a monitor that notifies the operator of the information. The notification unit 40 receives the maintenance signal and the identification signal transmitted from the controller 30 via wireless communication, and for example, the monitor displays various kinds of information on the basis of the received signal. Specifically, when the notification unit 40 receives the maintenance signal, the monitor displays the information prompting the maintenance of the inspection equipment 5 (specifically, replacement of the fluid). In addition, when the notification unit 40 receives the identification signal, the monitor displays the information indicating that the deterioration of the fluid has progressed significantly in the inspection of the fluid pressure cylinder 1 and information on the model number of the fluid pressure cylinder 1 that has caused the deterioration of the fluid.

As described above, in the state monitoring system 100, when the detected value from the fluid property sensor 10 is changed so as to exceed the predetermined value (in other words, when the detected value is changed significantly compared to the normal state), in order to identify the fluid pressure cylinder 1 corresponding to the timing at which the change has caused, the identification unit 34 can identify the fluid pressure cylinder 1 that has caused the deterioration of the fluid. As a result, it is possible to perform investigation of the upstream process of the inspection of the identified fluid pressure cylinder 1, and to specify any issues occurring in the upstream process. For example, it is possible to specify issues in the manufacturing process such as insufficient cleaning after machining of the fluid pressure cylinder 1, resulting in residual cutting fluid, insufficient drying after cleaning of the fluid pressure cylinder 1, resulting in residual cleaning liquid, or the like. Thus, by performing maintenance on the apparatus used for the operation, which has caused the issue or by encouraging workers who performed operation, which has caused the issue, to correct their working method, it is possible to solve the issue occurring in the upstream process of the inspection of the fluid pressure cylinder 1. As a result, it is possible to prevent the contaminant from entering the fluid pressure cylinder 1. If the state in which there is an issue in the upstream process of the inspection of the fluid pressure cylinder 1 continues, the progression of the deterioration of the fluid is accelerated. Thus, by solving the issue occurring in the upstream process of the inspection of the fluid pressure cylinder 1, it is possible to effectively prevent the deterioration of the fluid.

In addition, in a normal maintenance of the fluid, as described above, the fluid is deteriorated every time the fluid pressure cylinder 1 is inspected. Therefore, it is possible to efficiently monitor the deterioration of the fluid by monitoring the detected value from the fluid property sensor 10 at the timing at which the deterioration of the fluid progresses, in other words, the timing at which the fluid pressure cylinder 1 is exchanged. Thus, it is possible to perform the maintenance by predicting the deterioration of the fluid before the failure occurs in the inspection equipment 5.

Note that the identification unit 34 may output, to the notification unit 40, the identification signal including only the information on the model number of the identified fluid pressure cylinder 1 without including the information indicating that the deterioration of the fluid has progressed significantly in the identification signal.

In addition, the identification unit 34 may identify information other than the model number of the concerned fluid pressure cylinder 1 as long as the information can identify the fluid pressure cylinder 1 that has been being inspected when the detected value from the fluid property sensor 10 is changed. For example, the identification unit 34 may identify the time during which the concerned fluid pressure cylinder 1 is being inspected on the basis of the acquisition result from the exchange timing acquisition unit 33. As a result, the identification unit 34 can identify the concerned fluid pressure cylinder 1 from the viewpoint of the inspection time. In addition, in such a case, it is not essential for the inspection equipment 5 to output the inspection history of the fluid pressure cylinder 1 to the controller 30. The operator can compare the time during which the concerned fluid pressure cylinder 1 was inspected and the inspection history of the inspection equipment 5 by himself/herself and acquire the model number of the concerned fluid pressure cylinder 1.

According to the above-described embodiment, the following operational advantages are achieved.

In the state monitoring system 100, when the detected value from the fluid property sensor 10 is changed so as to exceed the predetermined value, in order to identify the fluid pressure cylinder 1 corresponding to the timing at which the change has caused, the identification unit 34 can identify the fluid pressure cylinder 1 that has caused the deterioration of the fluid. Therefore, it is possible to specify the cause of the deterioration of the fluid and to effectively prevent the deterioration of the fluid.

Following modifications are also within the scope of the present invention, and it is also possible to combine the configurations shown in the modifications with the configurations described in the above-mentioned embodiments, to combine the configurations described in the different embodiments described above with each other, and to combine the configurations described in the different modifications described below with each other.

### <First Modification>

In the above-mentioned embodiment, the exchange timing acquisition unit 33 of the controller 30 acquires the timing at which the fluid pressure cylinder 1 is exchanged on the basis of the temperature of the fluid detected by the temperature sensor provided on the detection unit 11 of the fluid property sensor 10. The method by which the exchange timing acquisition unit 33 acquires the timing at which the fluid pressure cylinder 1 is exchanged is not limited thereto. For example, the timing at which the fluid pressure cylinder 1 is exchanged may be acquired on the basis of the detected value from a pressure sensor that is provided on the flow paths 6a and 6b of the inspection equipment 5 and/or the flow paths 3a and 3b of the fluid pressure cylinder 1 (in other words, the pressure of the fluid). Specifically, during the inspection of the fluid pressure cylinder 1, the pressure of the fluid fluctuates due to the operation of the fluid pressure cylinder 1. On the other hand, when the fluid pressure cylinder 1 is exchanged, the pressure of the fluid does not substantially fluctuate. Thus, the exchange timing acquisition unit 33 detects that the fluid pressure cylinder 1 is being exchanged when a state in which the change in the pressure of the fluid detected by the pressure sensor is smaller than a predetermined threshold value continues for a predetermined time. In the above, the "predetermined threshold value" is set so as not to include a state in which the pressure of the fluid does not substantially fluctuate that occurs during the exchange of the fluid pressure cylinder 1, and the "predetermined time" is set to a long time so as not to include a time when the change in the pressure becomes small during the inspection of the fluid pressure cylinder 1 (for example, a time when extension and contraction of the fluid pressure cylinder 1 are switched).

In addition, in a case in which the inspection equipment 5 is the equipment in which all operations including the exchange of the fluid pressure cylinders 1 are automated, it may be possible to acquire the timing at which the fluid pressure cylinder 1 is exchanged by detecting operation of an actuator that exchanges the fluid pressure cylinders 1. Even with such a configuration, the same effects as those of the above-mentioned embodiment can be achieved.

### <Second Modification>

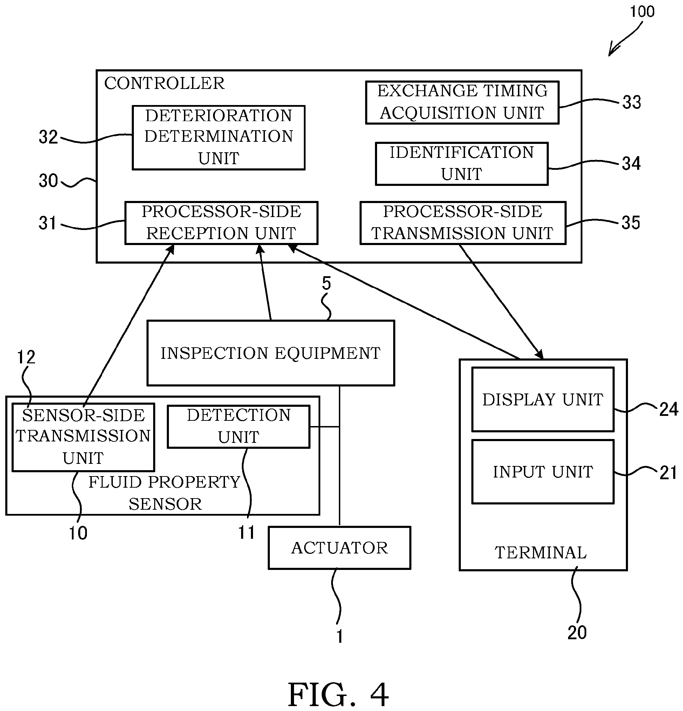

In the above-mentioned embodiment, the exchange timing acquisition unit 33 of the controller 30 acquires the timing at which the fluid pressure cylinder 1 is exchanged on the basis of the temperature of the fluid detected by the temperature sensor that is provided on the detection unit 11 of the fluid property sensor 10. In addition, the controller 30 notifies the operator of the information by the notification unit 40. The present invention is not limited thereto, and as shown in FIG. 4, the state monitoring system 100 may further include a terminal 20 to which exchange information indicating the timing at which the fluid pressure cylinder 1 is exchanged is input by the operator and which outputs the input exchange information to the exchange timing acquisition unit 33. The terminal 20 is provided instead of the notification unit 40.

The terminal 20 is, for example, a mobile device such as a smartphone, etc. or a personal computer, and is connected to the controller 30 wirelessly or via a wired connection. To the terminal 20, the exchange information is input by the operator using an input unit 21 such as a touch panel of the smartphone, a keyboard of the personal computer, and so forth, for example. An application for assisting the input of the exchange information is installed in the terminal 20, and the operator inputs the exchange information (the time at which the fluid pressure cylinder 1 is exchanged, the model number of the fluid pressure cylinder 1 exchanged, and so forth) to the input unit 21 according to the display of the application. The terminal 20 then outputs the input exchange information to the controller 30, and the exchange timing acquisition unit 33 of the controller 30 acquires the timing at which the fluid pressure cylinder 1 is exchanged on the basis of the exchange information input via the terminal 20.

Furthermore, the terminal 20 receives the maintenance signal and the identification signal from the controller 30 via the wireless communication, and a display unit 24 displays various kinds of information on the basis of the received signals. Specifically, when the terminal 20 receives the maintenance signal, the display unit 24 displays the information prompting the maintenance of the inspection equipment 5 (specifically, the replacement of the fluid). In addition, when the terminal 20 receives the identification signal, the display unit 24 displays the information indicating that the deterioration of the fluid has progressed significantly in the inspection of the fluid pressure cylinder 1 and the information on the model number of the fluid pressure cylinder 1 that has caused the deterioration of the fluid. With such a configuration, via the terminal 20, the operator can input the exchange information and notify the operator of the inspection target that is the cause of the deterioration of the fluid.

As long as the input exchange information input by the operator is input to the exchange timing acquisition unit 33 in the configuration, the terminal 20 is not essential. In addition, the exchange information may be acquired by monitoring the connection between the inspection equipment 5 and the fluid pressure cylinder 1 by the controller 30, etc. Furthermore, the terminal 20 may not output the exchange information to the controller 30, and it may be provided only to notify the operator of the information instead of the notification unit 40.

### <Third Modification>

In the above-mentioned embodiment, the electrical characteristic value of working fluid detected by the fluid property sensor 10 includes the dielectric constant and the conductivity, and the dielectric constant and the conductivity are increased as the working fluid is deteriorated with increased operating time of the fluid pressure cylinder 1, and are decreased when the working fluid is replaced with a fresh, non-deteriorated working fluid. The present invention is not limited thereto, and the electrical characteristic value of the working fluid detected by the fluid property sensor 10 may be a parameter other than the dielectric constant and the conductivity that is decreased as the working fluid is deteriorated. In this case, the concerned electrical characteristic value is increased when the working fluid is replaced with a fresh, non-deteriorated working fluid.

### <Fourth Modification>

In the above-mentioned embodiment, the controller 30 identifies, with the exchange timing acquisition unit 33 and the identification unit 34, the fluid pressure cylinder 1 corresponding to the timing at which the change has caused when the detected value from the fluid property sensor 10 is changed so as to exceed the predetermined value. The present invention is not limited thereto, and the processing performed by the exchange timing acquisition unit 33 and the identification unit 34 may be provided as a program for causing a computer to execute the processing. In other words, the program of the present modification is a program for causing the computer to execute the processing of the detected value input from the fluid property sensor 10 that detects the property of the inspection fluid by being provided on the inspection equipment 5 that performs the inspection by operating the fluid pressure cylinder 1, serving as the inspection target, which is sequentially exchanged with other fluid pressure cylinders 1, by supplying and discharging the inspection fluid to and from the fluid pressure cylinder 1. The program causes the computer to acquire the timing at which the fluid pressure cylinder 1 is exchanged, and when the detected value is changed so as to exceed the predetermined value, causes the computer to identify the fluid pressure cylinder 1 corresponding to the timing at which the change has caused on the basis of the timing at which the fluid pressure cylinder 1 is exchanged. In addition, for example, as described in the above-described second modification, the timing at which the fluid pressure cylinder 1 is exchanged is acquired from the exchange information input by the operator indicating the timing at which the fluid pressure cylinder 1 is exchanged.

The program for executing the series of processes described above is provided with a computer-readable storage medium. For example, the various programs to be executed by the computer may be stored in a non-transitory recording medium such as a CD-ROM, etc.

In addition, the various programs to be executed by the computer may be applications provided through a network.

The configurations, operations, and effects of the embodiments of the present invention configured as described above will be collectively described.

The state monitoring system 100 of the inspection equipment includes: the fluid property sensor 10 provided on the inspection equipment 5 for performing the inspection by operating the fluid pressure cylinder 1, serving as the inspection target, by supplying and discharging the inspection fluid to and from the fluid pressure cylinder 1 to be sequentially exchanged with other fluid pressure cylinder 1, the fluid property sensor 10 being configured to detect the property of the inspection fluid; the exchange timing acquisition unit 33 configured to acquire the timing at which the fluid pressure cylinder 1 is exchanged; and the identification unit 34 configured to identify, when the detected value from the fluid property sensor 10 is changed so as to exceed the predetermined value, the fluid pressure cylinder 1 corresponding to the timing at which the change has caused on the basis of the acquisition result from the exchange timing acquisition unit 33.

With this configuration, because the identification unit 34 identifies, when the detected value from the fluid property sensor 10 is changed so as to exceed the predetermined value, the fluid pressure cylinder 1 corresponding to the timing at which the change has caused, it is possible to identify the fluid pressure cylinder 1 that has been the cause of the deterioration of the inspection fluid. Therefore, by performing the investigation of the upstream process of the concerned fluid pressure cylinder 1 and by specifying the cause of the deterioration of the inspection fluid, it is possible to effectively prevent the deterioration of the inspection fluid.

In addition, the exchange timing acquisition unit 33 acquires the timing at which the fluid pressure cylinder 1 is exchanged from the exchange information indicating the timing at which the fluid pressure cylinder 1 is exchanged, the exchange information being input by the operator.

With this configuration, it is possible to acquire the timing at which the fluid pressure cylinder 1 is exchanged from the exchange information, which is input by the operator, and to identify the fluid pressure cylinder 1 that has been the cause of the deterioration of the inspection fluid.

In addition, the exchange timing acquisition unit 33 acquires the timing at which the fluid pressure cylinder 1 is exchanged from the temperature of the inspection fluid.

With this configuration, it is possible to acquire the timing at which the fluid pressure cylinder 1 is exchanged from the temperature of the inspection fluid and to identify the fluid pressure cylinder 1 that has been the cause of the deterioration of the inspection fluid.

In addition, the exchange timing acquisition unit 33 acquires the timing at which the fluid pressure cylinder 1 is exchanged from the pressure of the inspection fluid.

With this configuration, it is possible to acquire the timing at which the fluid pressure cylinder 1 is exchanged from the pressure of the inspection fluid and to identify the fluid pressure cylinder 1 that has been the cause of the deterioration of the inspection fluid.

In addition, a program is a program for causing a computer to execute processing of the detected value input from the fluid property sensor 10 for detecting the property of the inspection fluid by being provided on the inspection equipment 5, the inspection equipment 5 performing the inspection by operating the fluid pressure cylinder 1, serving as the inspection target, by supplying and discharging the inspection fluid to and from the fluid pressure cylinder 1, the fluid pressure cylinder 1 being sequentially exchanged with other fluid pressure cylinders 1. The program causes the computer to: acquire the timing at which the fluid pressure cylinder 1 is exchanged; and identify, when the detected value from the fluid property sensor 10 is changed so as to exceed the predetermined value, the fluid pressure cylinder 1 corresponding to the timing at which the change has caused on the basis of the timing at which the fluid pressure cylinder 1 is exchanged.

With this configuration, when the detected value from the fluid property sensor 10 is changed so as to exceed the predetermined value, because the fluid pressure cylinder 1 corresponding to the timing at which the change has caused is identified, it is possible to identify the fluid pressure cylinder 1 that has been the cause of the deterioration of the inspection fluid. Therefore, by performing the investigation of the upstream process of the concerned fluid pressure cylinder 1 and by specifying the cause of the deterioration of the inspection fluid, it is possible to effectively prevent the deterioration of the inspection fluid.

In addition, with the above-described program, it is characterized in that the timing at which the fluid pressure cylinder 1 is exchanged is acquired from the exchange information indicating the timing at which the inspection target is exchanged, the exchange information being input by the operator.

According to the present invention, it is possible to acquire the timing at which the fluid pressure cylinder 1 is exchanged from the exchange information, which is input by the operator, and to identify the inspection target that has been the cause of the deterioration of the inspection fluid.

Embodiments of the present invention were described above, but the above embodiments are merely examples of applications of the present invention, and the technical scope of the present invention is not limited to the specific constitutions of the above embodiments.

With respect to the above description, the contents of application No. 2022-52519, with a filing date of March 28, 2022 in Japan, are incorporated herein by reference.

## Claims

1. A state monitoring system of inspection equipment comprising:
a fluid property sensor provided on the inspection equipment for performing inspection by operating an inspection target by supplying and discharging inspection fluid to and from the inspection target, the inspection target being sequentially exchanged with other inspection targets, and the fluid property sensor being configured to detect a property of the inspection fluid;
an exchange timing acquisition unit configured to acquire a timing at which the inspection target is exchanged; and
an identification unit configured to identify, when a detected value from the fluid property sensor is changed so as to exceed a predetermined value, an inspection target corresponding to a timing at which the change has caused based on an acquisition result from the exchange timing acquisition unit.

2. The state monitoring system of the inspection equipment according to Claim 1, wherein
the exchange timing acquisition unit acquires the timing at which the inspection target is exchanged from exchange information indicating the timing at which the inspection target is exchanged, the exchange information being input by an operator.

3. The state monitoring system of the inspection equipment according to Claim 1, wherein
the exchange timing acquisition unit acquires the timing at which the inspection target is exchanged from temperature of the inspection fluid.

4. The state monitoring system of the inspection equipment according to Claim 1, wherein
the exchange timing acquisition unit acquires the timing at which the inspection target is exchanged from pressure of the inspection fluid.

5. A program for causing a computer to execute processing of a detected value input from a fluid property sensor for detecting a property of inspection fluid by being provided on inspection equipment, the inspection equipment performing an inspection by operating an inspection target by supplying and discharging the inspection fluid to and from the inspection target, and the inspection target being sequentially exchanged with other inspection targets, wherein
the program causes the computer to:
acquire a timing at which the inspection target is exchanged; and
identify, when the detected value is changed so as to exceed a predetermined value, an inspection target corresponding to a timing at which the change has caused based on the timing at which the inspection target is exchanged.

6. The program according to Claim 5, wherein
the timing at which the inspection target is exchanged is acquired from exchange information indicating the timing at which the inspection target is exchanged, the exchange information being input by an operator.
